Europäisches Patentamt

European Patent Office

Office européen des brevets

(11) Publication number : **0 498 562 A1**

# EUROPEAN PATENT APPLICATION

(21) Application number : **92300757.9**

(22) Date of filing : **29.01.92**

(51) Int. Cl.⁵ : **A61F 9/04**, A42B 1/20, A42B 1/06

(30) Priority : **04.02.91 US 650236**
**31.12.91 US 816331**

(43) Date of publication of application :
**12.08.92 Bulletin 92/33**

(84) Designated Contracting States :
**DE ES FR GB NL PT**

(71) Applicant : **Ulrich, Jan**
**1039 South Parker Road, Apartment No. 2F**
**Denver, Colorado 80231 (US)**

(72) Inventor : **Ulrich, Jan**
**1039 South Parker Road, Apartment No. 2F**
**Denver, Colorado 80231 (US)**

(74) Representative : **Foster, David Martyn et al**
**MATHISEN MACARA & CO. The Coach House**
**6-8 Swakeleys Road**
**Ickenham Uxbridge UB10 8BZ (GB)**

(54) Flexible visor-like head covering.

(57) A head covering is in the form of a visor-like cap (50) having an upper band (52) or head-encircling portion with a wire reinforcing member (53) around its upper edge and a visor (54) extending downwardly from the band (52) which includes a crescent-shaped section and a wire reinforcing member (56) embedded in the outer peripheral edge of the crescent-shaped section so as to cause the cap to assume the desired configuration when worn and when not worn to permit it to be coiled into a compact package for storage purposes.

FIG. 7

EP 0 498 562 A1

This invention relates to novel and improved visor-like caps or hats which can be coiled into a compact condition for convenient storage when not in use.

Visor-like caps are in widespread use for various out-door activities as a sunshade or screen. For instance, they are almost exclusively used by baseball players and by a great number of golfers and tennis players. Typically, the caps are subjected to an extreme amount of abuse, wear and tear as well as being deformed out of their proper configuration when laundered or folded into one's pocket or stuffed into a golf bag pocket. Under repeated use, conventional caps tend to become misshapen and this is especially true of the more popular form of visor in which the entire bill is reinforced with a cardboard or cardboard-like material which when folded or severely bent will not very easily return to its original curved configuration.

It has been proposed in the past to devise full-brimmed hats with outer wire or wire-like reinforcing members along their brims which can be coiled into a compact condition for storage purposes. Typically, such hats have required the use of some form of special material, such as, a fabric having directional strength or a particular dimensional relationship between the size of the brim and size of the reinforcing member. In this relation, it is desirable that the upper head-encircling portion be reinforced with a wire or wire-like member in such a way as to permit coiling into a compact storage condition as described; and when uncoiled and placed on the head will restore the upper portion of the cap to its original configuration.

Visor-like cap constructions have been devised with deformable reinforcing wires but are not designed in such a way that the cap can be coiled into a compact storage condition so as not to become misshapen when not in use; yet, when uncoiled, the visor will automatically spring back into its original crescent-shaped configuration with a curved bill when placed on the head of the wearer. Representative patents disclosing visor-like cap constructions with a reinforcing or stiffener section are U.S. Patent Nos. 2,931,046 to H. D. Klein, 1,666,098 to G. P. Kaul, 971,503 to C. I. Howard, 351,466 to J. J. Robbins and 1,435,533 to L. C. Knackstedt. Other foreign patents of interest are British Patent No. 187,553 to W. Schwalbe and Austrian Patent No. 3,627 to J. Komrowsky. A patent of particular interest in this regard is U.S. Patent No. 3,357,026 to R. C. Wiegandt in which a resilient stay or wire is designed to reinforce and lend a specific shape to the bill of a cap without utilizing a cardboard or similar material in the bill itself. However, in Weigandt, as is true in many of the other visor-like cap constructions, the resilient stay or stiffener member must be removed before the hat can be folded into a collapsed condition for storage.

Accordingly, it is an object of the present invention to provide for novel and improved headwear and particularly of the visor type which can be efficiently collapsed or coiled into a compact condition for storage when not in use.

It is another object of the present invention to provide for a novel and improved visor-type cap having a wire or wire-like reinforcing member which will establish the shape of the entire cap when worn and can be coiled with the cap into a compact package when not in use and will automatically return to its original shape or condition when placed on the head of the wearer.

A further object and feature of the present invention is to provide in a head covering for novel and improved reinforcing members which will serve as the sole means of shaping and support of the head covering and will remain a permanent part of the cap when laundered or folded for storage purposes without becoming misshapen; and further wherein the reinforcing members are so constructed and arranged that their characteristics will not be altered by laundering or cleaning of the cap, and will cause the cap to spring back to its desired shape when worn.

A still further object of the present invention is to provide for high strength, resilient reinforcing members for the bill and upper head-encircling portions of a cap which can be permanently inserted into the cap, are simple and inexpensive to manufacture and extremely rugged and durable in use.

In accordance with the present invention, a visor-like cap has been devised which is provided with the usual head-engaging or encircling portion, and a visor extending from the head-engaging portion includes an unreinforced, crescent-shaped section, and a wire-like reinforcing member extending around the outer peripheral edge of the crescent-shaped section, the reinforcing member including opposite ends terminating adjacent to said head-engaging portion, the reinforcing member being composed of a wire or wire-like material having a stiffness or straightness such that the visor will assume a generally convex cross-sectional configuration when placed on the head of a wearer and can be twisted into a coiled circular configuration of substantially reduced size in relation to its normal size when worn on the head of a wearer.

In a modified form of the present invention, the head-encircling portion is defined by an annular section having an upper terminal edge, a second reinforcing member is embedded in the terminal edge, the second reinforcing members also being composed of a wire or wire-like material having a memory and straightness such that when the cap is not worn it can be twisted into a coiled circular configuration of substantially reduced size in relation to its normal size when worn on the head of a wearer.

In the forms described, the reinforcing members are comprised of preformed wire rope; i.e., helical strands of wire wrapped into a single cable. Preferably, the rope is encased in an outer flexible plastic sheath; or the reinforcing members are polyurethane cords either in the form of a solid rod or a tubular member having a circular cross-section and of a stiffness corresponding to that of the cable member described. In this relation, when used in combination with a head-encircling portion having free, releasably connectable ends, most desirably the head-encircling portion and the crescent-shaped section are composed of an essentially shapeless material, such as, a soft fabric so that the reinforcing members can be twisted into a tight coil. The free ends of the head-encircling portion are then wrapped several times with the free ends reattached to retain the cap in a compact bundle or package which can be easily stored in one's pocket or in a small bag and occupy very little space; yet, when the ends are released, the reinforcing members are sufficiently resilient that they will spring back into their original configuration. A particularly important and favorable characteristic of the reinforcing members is that they exhibit no tendency to kink even when compressed into a tightly coiled condition.

Figure 1 is a perspective view of a preferred form of cap in accordance with the present invention;

Figure 2 is a front view in elevation illustrating the procedure for coiling the preferred form of cap shown in Figure 1 into a compact storage position;

Figure 3 illustrates the fully coiled configuration of the cap shown in Figures 1 and 2 for stowing in a bag;

Figure 4 is a cross-sectional view taken about lines 4-4 of Figure 1;

Figure 5 is another cross-sectional view of a modified form of reinforcing member incorporated into the bill of a cap;

Figure 6 is a perspective view of a modified form of cap with portions broken away to illustrate the construction of the bill of the cap;

Figure 7 is a perspective view of a modified form of cap in accordance with the present invention;

Figure 8 is a side view in elevation of the form of cap illustrated in Figure 7;

Figure 9 is a front view in elevation illustrating the procedure for coiling the modified form of cap shown in Figure 7 into a compact storage position;

Figure 10 illustrates the fully coiled configuration of the cap shown in Figures 7 to 9 for stowing in a bag;

Figure 11 is a cross-sectional view taken about lines 11-11 of Figure 7; and

Figure 12 is another cross-sectional view taken about lines 12-12 of Figure 7.

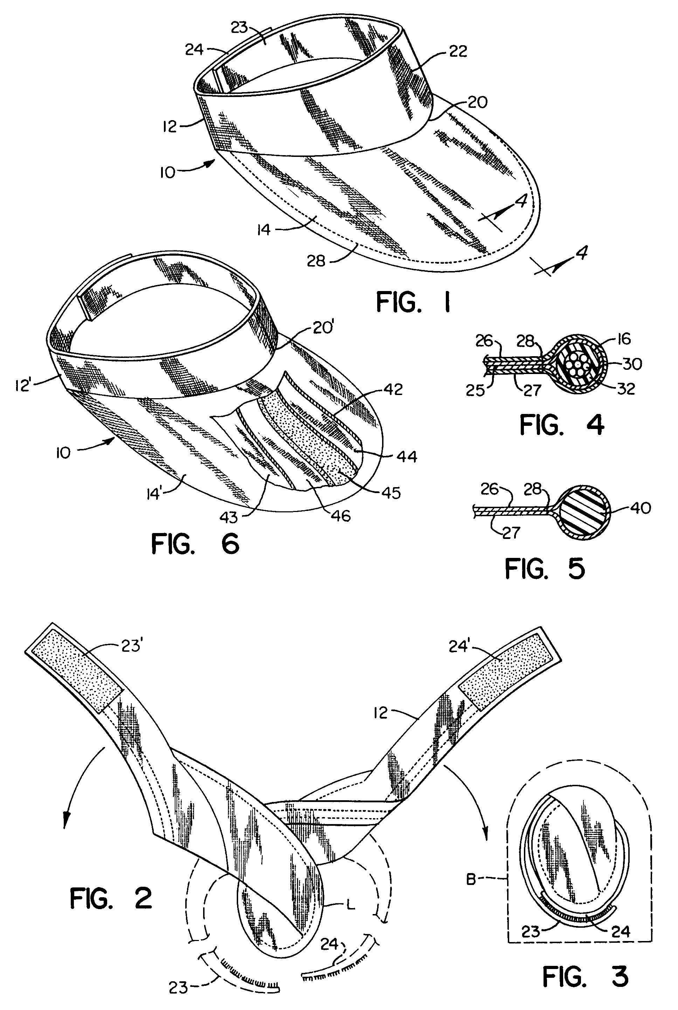

Referring in more detail to the drawings, there is illustrated in Figures 1 to 4 a preferred form of invention in the form of a cap 10 which is broadly made up of a head-encircling band 12 and a bill or visor 14 having a wire or wire-like reinforcing member 16 extending around the entire outer peripheral edge of the bill, an inner peripheral edge 20 of the bill being permanently attached to the band 12, such as, by suitable stitching, not shown. The style of cap 10 is given more as a setting for the present invention and is representative of numerous types of visor or cap constructions. For example, the cap may be in the form of a visor having no band 12 but which partially encircles the head, or a cap with a full crown to completely cover the head.

In the preferred form, the head-encircling band 12 is of standard construction comprising one or more layers of a fabric material defining a crown 22 above the visor 14, and opposite sides of the band 12 terminate in free ends 23 and 24 which are releasably attached together as shown. Typically, the free ends are releasably connected to one another by complementary Velcro fastening strips 23' and 24', as shown in Figure 2, along the facing surfaces of the free ends 23 and 24 so that the ends may be connected to establish the proper fit.

The visor or bill 14 is of generally crescent-shaped configuration and is suitably made up of upper and lower fabric layers 26 and 27, respectively, and an intermediate layer of cotton batting or muslin 25 or other flexible or soft fabric material; and the layers 25-27, as shown in Figure 4, are doubled over the reinforcing member 16 and seamed together as at 28 to closely surround and securely retain the reinforcing member 16 in position. An important feature is the ability of the reinforcing member 16, by virtue of its composition and characteristics, to operate as the exclusive means of support and shaping of the bill when worn so as to be of generally upwardly convex configuration in a direction circumferentially across the inner peripheral edge 20, and the degree of convexity is progressively reduced in a radially outward direction toward the outer peripheral edge of the bill.

The reinforcing member 16 traverses the entire extent of the outer peripheral edge of the bill 14 and is preferably comprised of a preformed wire rope or helical strands of wire 30, and the strands are wrapped together and encased within a plastic sheath 32. An excellent reinforcing member is that referred to as tiller cable and which is made up of galvanized or stainless steel wire strands coated with a vinyl or other plastic material. In the form shown, a 7 x 7 3/32" galvanized steel wire is coated with vinyl to 3/16", such as, Part No. 51820 manufactured and sold by Tie Down Engineering of Atlanta, Georgia. The wire strands are wrapped in the form of a helix which when encased within a vinyl sheath as at 32 has sufficient resiliency that it will bend into the generally crescent-shaped or arcuate configuration as illustrated in Figure 1 when the cap 10 is placed in an encircling position around the head of the wearer. By forming the strands together into a single helix 30, the member 16 will not bend as easily as a single strand and will effectively resist any tendency to kink or crease when folded or coiled.

The inner peripheral edge of the bill 14 when secured to the band 12 will lend additional support or reinforcement to the cap when placed on the head, but the major reinforcement and support is provided by the member 16 and to the extent that the crescent-shaped layers 25, 26 and 27 require no other shaping or supporting means, such as, cardboard, plastic or the like customarily used in conventional visor or cap constructions. In this relation, the reinforcing member, being constrained to follow the curved outline of the outer peripheral edge of the bill possesses a sufficient degree of straightness or memory, that it will exert a slight degree of tension on the fabric material of the bill so as to maintain it in a taut or stretched condition.

As further illustrated in Figures 2 and 3, the shapeless characteristics of the bill 14 when combined with the limited resiliency of the reinforcing member 16 permit convenient folding or collapsing of the cap 10 into a compact package when not in use. Thus, by disengaging the free ends 23 and 24, the brim or outer peripheral edge of the bill 14 is twisted into a small loop or coil as designated at L. The free ends 23 and 24 of the band 12 are then wrapped around the loop L, as shown in Figure 2, with the free end 23 facing in and the free end 24 facing out so that upon completion of wrapping can be secured together. This wrapping may take place or be done in a single plane in surrounding relation to the loop or by wrapping over and under the loop while maintaining the loop in a tightly coiled condition. For instance, the free end 24 as shown in Figure 2 would be wrapped in a clockwise direction around the loop, followed by wrapping the end 23 in an opposite direction, counterclockwise around the loop L and the partially wrapped free end 23 until the free end 24 moves into mating engagement with the end 23, essentially as shown in the completed condition in Figure 3. Figure 3 also illustrates a typical manner of storage of the cap by inserting the wrapped cap into a small bag or pouch B.

In the modified form shown in Figure 6, like parts are correspondingly enumerated with prime numerals to those of Figures 1 to 4 and comprises a cap 10′ having a band 12′ and visor or bill 14′. As before, the inner peripheral edge 20′ of the visor is permanently attached to the band 12′; however, the visor portion 14′ consists of an upper fabric layer 42 and a lower fabric layer 43 separated by intermediate layers consisting of equal thicknesses of a heavyweight interfacing layer 44, layer of cotton batting or muslin 45 and another heavyweight interfacing layer 46.

A reinforcing member 40, as shown in Figure 5, extends continuously around the outer peripheral edge between opposite sides of the bill 14′ at their points of attachment to the band 12′. The modified form of reinforcing member 40 has characteristics comparable to those of the reinforcing member 16 as described but is composed of an extruded plastic cord and preferably composed of a polyurethane material. When the cord is in the form of a solid rod, the durometer or hardness would be in the range of 50 to 60 shore and when of tubular configuration would be more in the range of 80 to 90 shore. The diameter of the cord is typically in the range of 1/8″ to 3/16″, and the same would be true of a tubular cord member. Either type of cord has sufficient resiliency or straightness that when inserted into the outer curved peripheral edge of the bill 14 will be under a certain amount of tension tending to stretch the layers of material 42 to 46 so as to be taut and assume a generally convex configuration as previously described in the preferred form of Figures 1 to 4. The tension in the cord 40 is further increased by twisting or coiling into the storage position as described with reference to the preferred form of reinforcing member 16 in Figures 2 and 3.

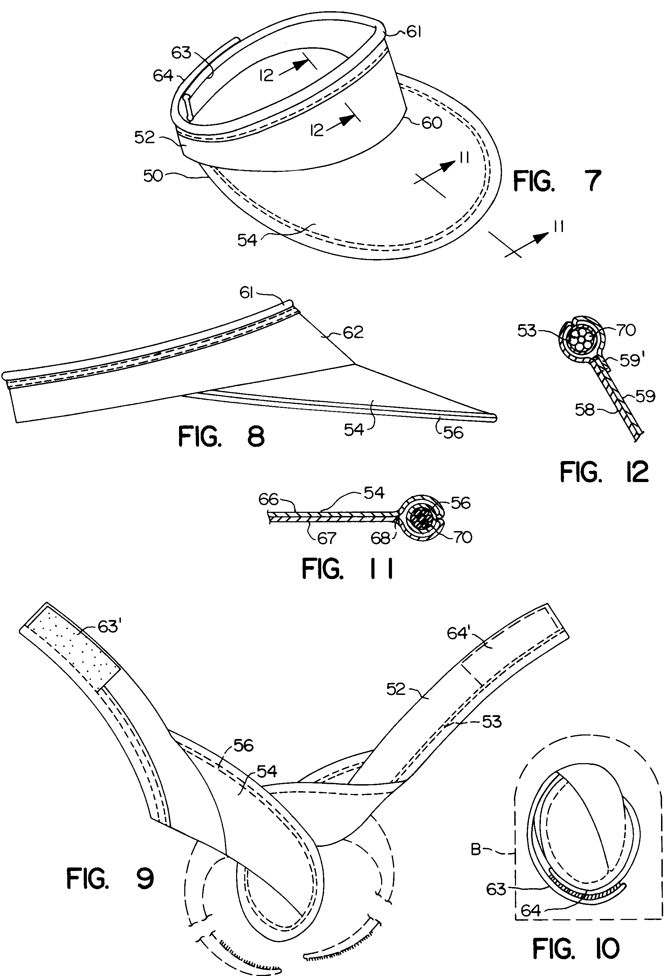

In Figures 7 to 12 the modified invention comprises a cap 50 with a head-encircling band 52 having a wire or wire-like reinforcing member 53 extending around its upper peripheral edge and a bill or visor 54 having a wire or wire-like reinforcing member 56 extending around the entire outer peripheral edge of the bill, an inner peripheral edge 60 of the bill being permanently attached to the band 52, such as, by suitable stitching, not shown.

The head-encircling band 52 comprises inner and outer layers 58 and 59 of a fabric material defining a crown 62 above the visor 14 and terminating in an upper edge 61; and opposite sides of the band 52 taper rearwardly away from the crown 62 and terminate in free ends 63 and 64 which are releasably attached together as shown. Preferably, the inner layer 58 has its upper edge doubled over the reinforcing member 53 and seamed to the outer layer 59 as designated at 59′ so as to surround and securely retain the reinforcing member 53 in position around the upper peripheral edge 61. Typically, the free ends 63 and 64 are releasably connected to one another by complementary Velcro fastening strips 63′ and 64′, as shown in Figure 9, along the facing surfaces of the free ends 63 and 64 so that the ends may be connected to establish the proper fit.

The visor 54 is of generally crescent-shaped configuration and is suitably made up of upper and lower fabric layers 66 and 67, respectively, and an intermediate layer of cotton batting or muslin 65 and the layers 65-67, as shown in Figure 10, are doubled over the reinforcing member 56 and seamed together as at 68 to closely surround and securely retain the reinforcing member 56 in position. The reinforcing members 53 and 56, by virtue of their composition and characteristics, operate as the exclusive means of support and shaping of the band 52 and bill 54 when worn. Accordingly, when the ends 63 and 64 are connected together as described, the upper reinforcing member 53 will assume a generally circular or annular configuration so as to cause the band 52 not only to assume the same annular configuration but to lend a degree of stiffness to the band in a vertical direction, as illustrated in Figure 8, to prevent the upper edge 61 from sagging or otherwise becoming misshapen. In a similar manner, the bill 54 will assume a generally upwardly convex configuration in a direction circumferentially across the inner peripheral edge 60, and the degree of convexity is progressively reduced in a radially outward direction toward the outer peripheral edge of the bill 54.

Again, the reinforcing members 53 and 56 are preferably comprised of a preformed wire rope or helical strands of wire 70 as described in Figures 1 to 6. In a like manner to the reinforcing member 56, the reinforcing member 53 will maintain the fabric material of the band 52 in a somewhat stretched condition so as to avoid wrinkling or drooping of the band.

As further illustrated in Figures 9 and 10, the shapeless characteristics of the band 52 and bill 54 when combined with the limited resiliency of the reinforcing members 53 and 56 permit convenient folding or collapsing of the cap 50 into a compact package when not in use. Thus, by disengaging the free ends 63 and 64, the brim is twisted into a small loop or coil as designated at L. The free ends 63 and 64 of the band 52 are then wrapped around the loop L, as shown in Figure 9, with the free end 63 facing in and the free end 64 facing out so that upon completion of wrapping can be secured together. This wrapping may be done in a single plane in surrounding relation to the loop or by wrapping over and under the loop while maintaining the loop in a tightly coiled condition. For instance, the free end 64 as shown in Figure 9 would be wrapped in a clockwise direction around the loop, followed by wrapping the end 63 in an opposite direction, counterclockwise around the loop L and the partially wrapped free end 63 until the free end 64 moves into mating engagement with the end 63, essentially as shown in the completed condition in Figure 9. Figure 10 also illustrates a typical manner of storage of the cap by inserting the wrapped cap into a small bag or pouch B.

It is therefore to be understood that various other modifications and changes may be made in the construction and arrangement of elements comprising the preferred and modified forms of invention as well as the composition of materials utilized without departing from the spirit and scope of the present invention as defined by the appended claims and reasonable equivalents thereof.

## Claims

1. In a head covering, a visor-like cap provided with a flexible head-encircling portion, the improvement comprising:

   a visor extending from said head-encircling portion including an unreinforced, flexible crescent-shaped section, and an elongated reinforcing member embedded in an outer peripheral edge of said crescent-shaped section, said reinforcing member including opposite ends terminating adjacent to said head-encircling portion, said reinforcing member composed of a wire or wire-like material having a resiliency such that said visor will assume a generally convex configuration when placed on the head of a wearer and when not worn said visor including said reinforcing member can be twisted into a tightly coiled, loop-shaped configuration of substantially reduced size in relation to its normal size when worn on the head of a wearer.

2. In a head covering according to claim 1, said visor being characterized by being manually twisted without kinking into a circular coil with said reinforcing member coiled through greater than 360° into a coiled portion with said opposite ends of said reinforcing member overlapping said coiled portion.

3. In a head covering according to claim 1, said head-encircling portion having free, releasably connectable ends which when said visor is twisted into a coil are wrapped around said coil.

4. In a head covering according to claim 1, said reinforcing member comprised of helical strands of wire and a flexible sheath encircling said wire.

5. In a head covering according to claim 4, said wire being a galvanized steel wire.

6. In a head covering according to claim 1, said reinforcing member being in the form of a plastic cord having a circular cross-section.

7. In a head covering according to claim 6, said cord being in the form of a solid rod having a durometer in the range of 50 to 60 shore.

8. In a head covering according to claim 6, said cord being in the form of tubing having a durometer in the range of 80 to 90 shore.

9. In a head covering wherein a cap is provided with a flexible head-encircling portion, the improvement comprising:

   a visor extending from said head-encircling portion including an unreinforced crescent-shaped section composed of a shapeless material, such as, a soft fabric, and reinforcing means in the form of an elongated stiffener member embedded in an outer peripheral edge of said crescent-shaped section and traversing the length thereof, said stiffener member including opposite ends terminating adjacent to said head-encircling portion, said crescent-shaped section surrounding said stiffener at its outer peripheral edge, said stiffener member composed of a resilient wire or wire-like material having a tendency to straighten itself such that said reinforcing means will cause said visor to be stretched into an upwardly convex configuration when placed on the head of a wearer and when not worn said visor including said reinforcing member can be twisted into a tightly coiled circular or loop-shaped configuration of substantially reduced size in relation to its normal size when worn on the head of a wearer.

10. In a head covering according to claim 9, said head-encircling portion having free, releasably connectable ends which when said visor is twisted into a coil are wrapped in circular fashion in overlapping relation to one another and in surrounding relation to said visor.

11. In a head covering according to claim 10, said visor having upper and lower layers of a soft fabric material, said stiffener interposed between said upper and lower layers, and a seam joining said upper and lower layers together in closely surrounding relation to said stiffener along said outer peripheral edge of said visor.

12. In a head covering according to claim 9, said stiffener member composed of a tiller cable having strands of wire and a flexible sheath encircling said strands.

13. A cap adapted for use as a head covering comprising in combination an upper, flexible head-encircling portion and a visor extending downwardly from said head-encircling portion, said visor including an unreinforced, flexible crescent-shaped section and a first elongated reinforcing member embedded in an outer peripheral edge of said section, said first reinforcing member including opposite ends terminating adjacent to said head-encircling portion, said head-encircling portion defined by an annular section having an upper peripheral edge, a second reinforcing member embedded in said upper peripheral edge, said first and second reinforcing members each composed of a wire or wire-like material having a straightness and memory such that when said cap is not worn said cap can be twisted into a tightly coiled, loop-shaped configuration of substantially reduced size in relation to its normal size when worn on the head of a wearer.

14. A cap according to claim 13, said second reinforcing member having a memory such that said head-encircling portion will assume a generally upright disposition above said visor when worn on the head of a wearer.

15. A cap according to claim 14, said head-encircling portion and said visor being capable of being manually twisted without kinking into a circular coil with said first and second reinforcing members coiled through greater than 360° into a coiled portion and with said opposite ends of said first and second reinforcing members overlapping said coiled portion.

16. A cap according to claim 13, said head-encircling portion having free, releasable connecting ends which when said visor is twisted into a coil are wrapped around said coil.

17. A cap according to claim 13, said first and second reinforcing members comprised of helical strands of wire, and a flexible sheath encircling said strands of wire.

18. A cap according to claim 17, said wire being a galvanized steel wire.

19. A cap according to claim 13, said crescent-shaped section and said upper head-encircling portion each composed of a shapeless material, such as, a soft fabric.

20. A cap according to claim 19, said head-encircling portion including inner and outer layers of a soft fabric material, said second reinforcing member interposed between said inner and outer layers, and a seam joining said inner and outer layers together in closely surrounding relation to said second reinforcing member.

21. A hat comprising in combination an upper, flexible head-encircling portion and a brim inclining downwardly from said head-encircling portion, said brim including an annular section and a first elongated reinforcing member embedded in an outer peripheral edge of said annular section, said first reinforcing member completely encircling said brim, said head-encircling portion defined by an upright annular section having an upper peripheral edge, a second reinforcing member embedded in said upper peripheral edge, said first and second reinforcing members each composed of a wire or wire-like section having a straightness and memory such that when said hat is not worn said hat can be twisted into a tightly coiled, loop-shaped configuration of substantially reduced size in relation to its normal size when worn on the head of a wearer, and said second reinforcing member having a memory such that said head-encircling portion will assume a generally upright disposition above said visor when worn on the head of a wearer.

22. A hat according to claim 21, said head-encircling portion and said brim being capable of being manually twisted without kinking into a circular coil with said first and second reinforcing members coiled through greater than 360° into a coiled portion with said opposite ends of said first and second reinforcing members overlapping said coiled portion.

23
24
22
12
20
10
14
4
28
4

**FIG. 1**

20'
42
12'
44
10
45
14'
46
43

**FIG. 6**

26
28
16
25
30
27
32

**FIG. 4**

26
28
27
40

**FIG. 5**

23'
24'
12
B
23
24
L
24
23

**FIG. 2**

**FIG. 3**

FIG. 7

FIG. 8

FIG. 12

FIG. 11

FIG. 9

FIG. 10

**European Patent Office**

# EUROPEAN SEARCH REPORT

Application Number

EP 92 30 0757

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | US-A-3 357 026 (WIEGANDT) <br> * column 2, line 16 - line 39; figures * <br> --- | 1-6,9 | A61F9/04 <br> A42B1/20 <br> A42B1/06 |
| Y <br> A | CH-A-666 164 (ROETHLIN) <br> * abstract; figures * <br> --- | 1-6,9 <br> 21 | |
| A | US-A-4 393 519 (NICASTRO) <br> * column 5, line 27 - line 34; figure 9 * <br> --- | 1 | |
| A | US-A-4 481 680 (MASON ET AL.) <br> * column 2, line 14 - line 35; figures 1-3 * <br> --- | 13 | |
| A | GB-A-347 990 (CRETIN-BILLET ET FILS, S.L.) <br> ----- | | |

TECHNICAL FIELDS SEARCHED (Int. Cl.5)

A61F
A42B

The present search report has been drawn up for all claims

| Place of search | Date of completion of the search | Examiner |
|---|---|---|
| THE HAGUE | 26 MARCH 1992 | SANCHEZ Y SANCHEZ J. |

EPO FORM 1503 03.82 (P0401)